# EUROPEAN PATENT SPECIFICATION

(11) **EP 1 789 582 B1**
(45) Date of publication and mention of the grant of the patent: **06.10.2010**
(21) Application number: 05772474.2
(22) Date of filing: 05.08.2005
(51) Int. Cl.: C12Q 1/68

(54) **NEISSERIA GONORRHOEAE DETECTION USING THE 5' UNTRANSLATED REGION OF THE OPA GENE**
NACHWEIS VON NEISSERIA GONORRHOEAE UNTER VERWENDUNG DES 5'-NICHTTRANSLATIERTEN BEREICHS DES OPA-GENS
DÉTECTION DE NEISSERIA GONORRHOEAE UTILISANT LA RÉGION 5' NON-TRADUITE DU GÈNE OPA

(30) Priority: 05.08.2004 EP 04077241
(43) Date of publication of application: 30.05.2007
(73) Proprietor: Jeroen Bosch Ziekenhuis, 5211 RW 's-Hertogenbosch (NL)
(72) Inventor: HERMANS, Maria, Hendrika, Anna, NL-5251 DB Vlijmen (NL)
(74) Representative: van Loon, C.J.J.
(86) International application number: PCT/NL2005/000574
(87) International publication number: WO 2006/014109

(56) References cited:
- EP-A2- 0 404 161
- EP-A2- 0 919 633
- WO-A2-2004/043227
- US-A- 5 427 930
- DATABASE EMBL [Online] 2 April 1988 (1988-04-02), "Neisseria gonorrhoeae gene for protein II (pFLOB709)" XP002312655 retrieved from EBI accession no. EM_PRO:NGPIIG2 Database accession no. NGPIIG2
- WHILEY, BUDA, BAYLISS, COVER, BATES AND SLOOTS: "A new confirmatory Neisseria gonorrhoeae real-time PCR assay targeting the porA pseudogene" EUR. J. CLIN. MICROBIOL. INFECT. DIS., vol. 23, 10 July 2004 (2004-07-10), pages 705-710, XP002315780
- WHILEY DAVID M ET AL: "A real-time PCR assay for the detection of Neisseria gonorrhoeae by LightCycler" DIAGNOSTIC MICROBIOLOGY AND INFECTIOUS DISEASE, vol. 42, no. 2, February 2002 (2002-02), pages 85-89, XP002315781 ISSN: 0732-8893
- O' ROURKE, ISON, RENTON, SPRATT: "Opa-typing: a high-resolution tool for studying the epidemioloy of gonorrhoeae" MOLECULAR MICROBIOLOGY, vol. 17, no. 5, 1995, pages 865-875, XP002315782 cited in the application

## Description

The invention relates to microbiology and molecular diagnostics. More specifically, the invention relates to the specific and sensitive detection of *Neisseria gonorrhoeae* (*NG*) in clinical samples.

*Neisseria gonorrhoeae* is the second most prevalent sexually transmitted bacterial infection after *Chlamydia trachomatis.* A significant proportion of the infections, especially in women, are asymptomatic. If they remain undiscovered, they may result in spread to sexual partners and long-term consequences such as pelvic inflammatory disease, chronic pelvic pain, ectopic pregnancy, neonatal conjunctivitis, and infertility (12). Therefore, accurate diagnosis of both symptomatic and asymptomatic infections is critical.

A number of techniques have been developed to detect genital infections caused by *NG.* The current "golden standard" for diagnosis of infection is by culturing on selective media. However, even under optimal laboratory conditions, the sensitivity of gonococcal cultures ranges from 85 to 95% for acute infection (23) and falls to approximately 50% for females with chronic infections (2) due to poor specimen collection, transport and storage. Known molecular biological methods for the detection of *NG* include nucleic acid amplification-based techniques, including the ligase chain reaction (LCR), strand displacement amplification assay and PCR (1, 9, 14, 17, 19, 24, 28). Whereas these methods have been shown to display both high sensitivity and specificity, each of these tests has limitations including variable sensitivities to inhibitors; cross-reactivity with other micro-organisms, limited throughput, high costs and dedicated equipment. For example, the Cobas Amplicor test for *NG* (Cobas Amplicor CT/NG; Roche Molecular Systems, Branchburg, N.J.) produces false-positive results with certain strains of *N. subflava, N. cinerea* and *Lactobacillus,* and a subsequent conformation test is necessary (11, 13, 22, 29). CppB-and 16S rRNA-gene based assays are used for confirmation, however, 5 to 6% of *NG* strains do not carry the CppB-plasmid (6), and not all 16S rRNA-based tests are sensitive and specific enough (11, 30).

The LCx® *NG* Assay (Abbott Laboratories, Abbott Park, IL) uses the LCR nucleic acid amplification method to detect the presence of *NG* DNA directly in clinical specimens. The four oligonucleotide probes in the LCx assay recognize and hybridize to a specific target sequence within the *Opa* genes of *NG* DNA (see US 5,427,930). The oligonucleotides are designed to be complementary to the target sequence in such a way that in the presence of *NG* target, the probes will bind adjacent to one another. They can then be enzymatically joined to form the amplification product that subsequently serves as an additional target sequence during further rounds of amplification. The product of the LCR reaction is detected on the Abbott LCx Analyzer.

All meningococcal and gonococcal strains express opacity (opa) proteins, so called because of their contribution to colony opacity during growth of the bacteria on agar plates (26). They are a family of basic integral outer membrane proteins of approximately 27 kDa. Eleven to thirteen individual *opa* genes have been identified in *NG,* whereas N. meningitides have less (three or four) *opa* genes. Opa-like proteins are expressed in a number of commensal *Neisseriaceae* as well (27). The *opa* genes in *NG* are contained in separate loci (*opaA* through -*K*) (4) and are subject to on/off phase variation. Changes in the repetitive sequence within the various *opa* loci result in this variable expression in a single bacterium (25). Opa expression has been found to promote gonococcal adherence to epithelial cells, entry into epithelial cells via binding to cell surface proteoglycans (3, 8, 16, 31, 32) and gonococcal interactions with polymorphonuclear leukocytes (15). Furthermore, opa expression enhances resistance against complement-mediated killing (5).

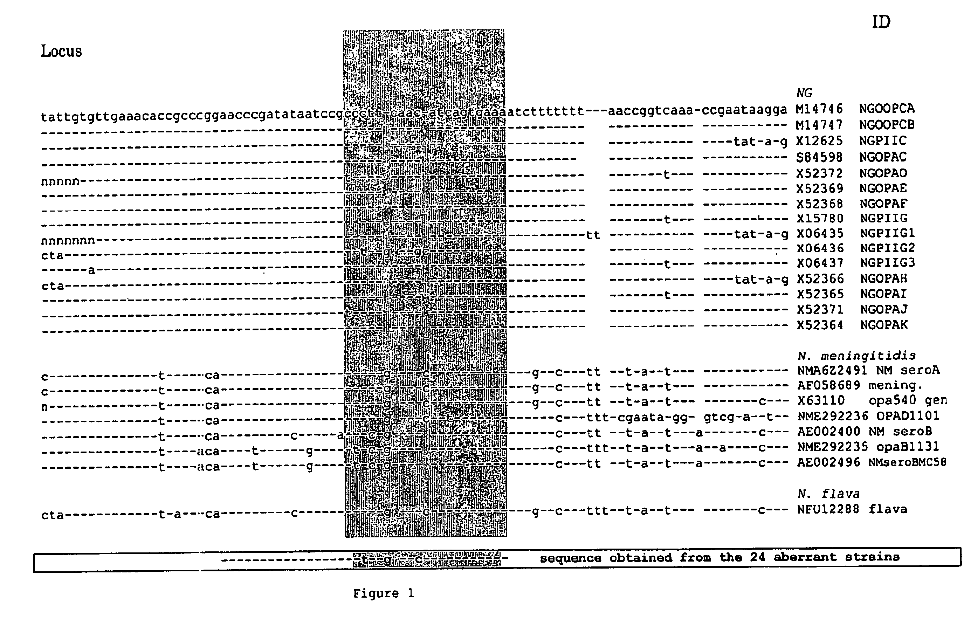

Because the *opa* genes are multicopy genes that harbour conserved regions and encode proteins with physiological functions, the present inventors considered them as suitable target sequences for a real time PCR amplification assay and set out to develop a specific and sensitive PCR-based assay for the detection of NG.
Sequences covering a conserved region within the 5' untranslated region of the *opa*genes were obtained from the NCBI database. Based on homology, sequences of *NG*, *N. meningitides* and *N. flava* were retrieved and aligned in Figure 1. Primers and a minor groove binding (MGB) probe opa-1 (Table 1) were designed and adapted to TaqMan-standards using Primer Express software (Applied Biosystems). The forward PCR primer used in the invention (see Table 1) partially overlaps with LCR probe 66.1 disclosed in US 5,427,930. Oligonucleotide probe opa-1 according to the invention overlaps with the five nucleotides at the 3' end of LCR probe 66.3 of US 5,427,930. To optimize the *opa*-based *NG* PCR assay, a panel of 448 clinical *NG* strains (see Materials and Methods) was tested. Of these 448 strains, 424 generated a positive fluorescent signal in the TaqMan-PCR employing probe *opa*-1, and 24 did not. However, when analysing the PCR products of these 24 "aberrant" *NG* strains on agarose gel, all 24 showed ample PCR products of the expected size. Apparently, the amplified PCR products (amplicons) of the "aberrant" strains were not detected by probe *opa*-1. Surprisingly, sequence analysis of the amplicons that were generated by the primer set but which were not detected by the *opa*-1 probe revealed exactly the same sequence for all 24 aberrant *NG* strains (indicated in Figure 1). This sequence is characterized by the presence of a nucleotide sequence 5'-TTTGAACC-3', which is not present in any of the 5' untranslated regions of the known *opa* genes obtained from the NCBI database, based on which probe *opa*-1 was designed. The sequence of the aberrant strains shows two mismatches and one insertion compared to the sequence of the *opa*-1 probe, which is apparently sufficient to prevent the probe from hybridising to the amplified target sequence i.e. the region from nucleotide at position 58 to position 40 located 5' of the start codon of a gene encoding an opacity protein of *NG.*

Therefore, a novel probe (*opa*-2) was designed to detect the amplified sequence of these aberrant NG strains. Provided is a NG-specific oligonucleotide, comprising a nucleotide sequence 5'-TTTGAACC-3', or its complement, capable of hybridising to the 5'-untranslated region of an *opa*-gene of NG encompassing nucleotides 57 to 50 located 5' of the NG *opa* start codon (further referred to as target sequence) or its complement. This means that one nucleotide within the stretch of eight nucleotides may be substituted by another nucleotide provided that the oligonucleotide can still recognize and bind to its target sequence. Preferably, the substitution does not involve substitution of the T residue at the 5'- end, the 3'- end C residue or the G residue, as these residues represent the mismatches with the *opa*-1 probe and are likely responsible for recognition of the aberrant NG strains.

A complementary sequence 5'-tcagtgatggttcaaagttc-3' is known from US 6,617,162. There it has been used as a primer targeting the human estrogen receptor alpha RNA. Thus it can be seen as an accidental anticipation for the present invention.

The term "oligonucleotide" refers to a short sequence of nucleotide monomers joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). The length of the oligonucleotide can vary. Generally speaking, the chance that a hybrid is formed between an oligonucleotide and a complementary target nucleic acid sequence increases with increasing length of the oligonucleotide. On the other hand, the specificity of hybrid formation decreases with increased length of the oligonucleotide. An oligonucleotide of the invention is preferably 14 to 40 nucleotides in length, more preferably 16 to 30 nucleotides, most preferably 18 to 22 nucleotides. The characterizing sequence 5'-TTTGAACC-3' is preferably flanked at both the 5' and the 3' end by at least one nucleotide complementary to the 5' untranslated region an NG *opa* gene.

A preferred oligonucleotide according to the invention is 5'-CTTTGAACCATCAGTGAAA-3' or its complement. As is illustrated in the Examples, this oligonucleotide is advantageously used as detection probe to detect aberrant strains of NG in a real-time PCR assay.

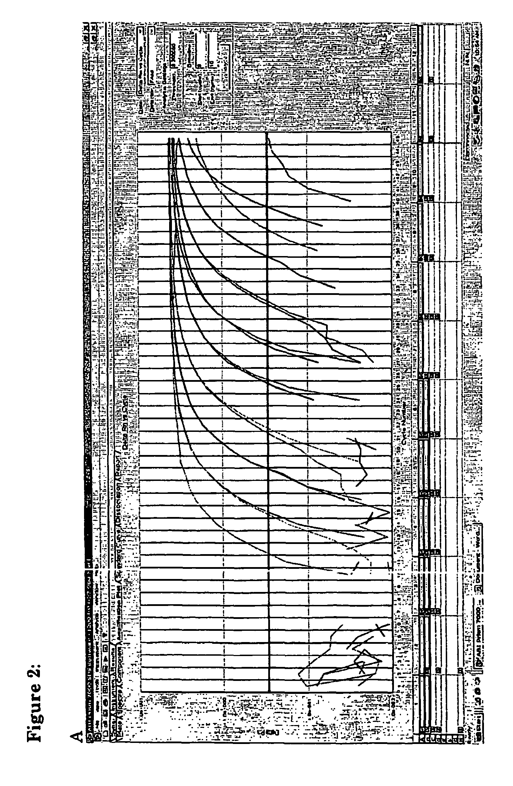

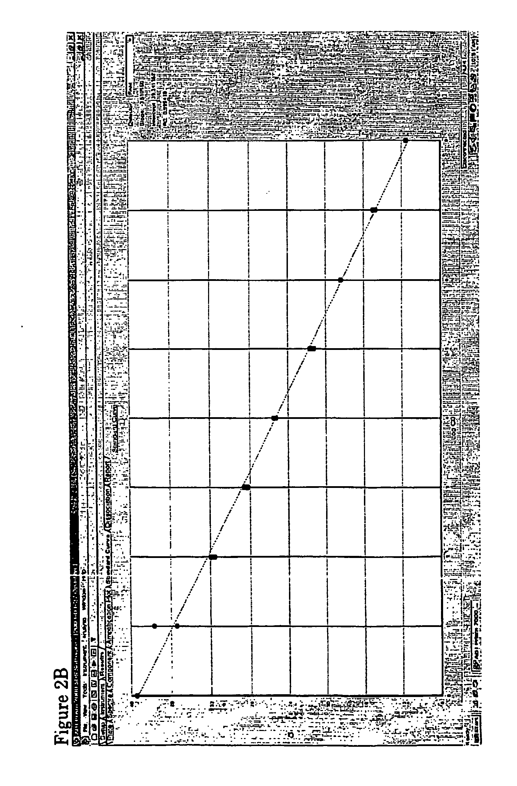

When the specificity of the *opa*-2 probe was investigated by testing a panel of non NG microorganisms, including DNA from 12 different other Neisseriaceae, no positive signal was detected when using probes *opa*-1 and *opa*-2 (see item 2.3 in the Example below). Sensitivity assays (see item 2.4 in the Example below) revealed that NG DNA could be measured linearly over a range of 8 log scales (see Fig. 2). One femtogram of NG DNA was detectable in the majority of the samples tested. Thus, herewith the invention provides an oligonucleotide probe for the specific and sensitive detection of NG strains, which remained undetected using a PCR probe based on the opa sequence of known NG strains. The aberrant NG strains detected by a nucleotide probe of the invention may be detected using the 16S rRNA test or the CppB test. However, as said above, these tests are usually not sensitive and specific enough for clinical application.

An oligonucleotide probe according to the invention can be labeled with any label known in the art of nucleic acid chemistry. In one embodiment, an oligonucleotide of the invention comprises one or more detectable labels. Detectable labels or tags suitable for use with nucleic acid probes are well-known to those of skill in the art and include, but are not limited to, radioactive isotopes, chromophores, fluorophores, chemiluminescent and electrochemiluminescent agents, magnetic labels, immunologic labels, ligands and enzymatic labels. Preferably, an oligonucleotide comprises a chromophore or fluorescent label, as these can generally be easily detected with high sensitivity and specificity. Examples of fluorescent labels are fluoresceins, rhodamines, cyanines, phycoerythrins, and other fluorophores known to those of skill in the art, including 6-FAM, HEX, JOE, TET, ROX, TAMRA, Fluorescein, Cy3, Cy5, Cy5.5, Texas Red, Rhodamine, Rhodamine Green, Rhodamine Red, 6-CarboxyRhodamine 6G, Oregon Green 488, Oregon Green 500, Oregon Green 514 and 6-CarboxyRhodamine 6G.

In one embodiment of the present invention, an NG-specific oligonucleotide comprises a fluorescent label (fluorophore) and/or a fluorescence quenching agent. In a preferred embodiment, an oligonucleotide of the invention contains both a fluorophore and a quenching agent. An oligonucleotide comprising such a fluorophore/quencher pair is suitably used as TaqMan™ probe in a real time PCR assay (see also below). Quenching agents are those substances capable of absorbing energy emitted by a fluorophore so as to reduce the amount of fluorescence emitted (i.e., quench the emission of the fluorescent label). Different fluorophores are quenched by different quenching agents. In general, the spectral properties of a particular fluorophore/quenching agent pair are such that one or more absorption wavelengths of the quencher overlaps one or more of the emission wavelengths of the fluorophore. Examples of quenching agents are TAMRA, DABCYL, BHQ-1 and BHQ-2.

A preferred fluorophore/quencher pair is fluorescein/tetramethylrhodamine; additional fluorophore/quencher pairs can be selected by those of skill in the art by comparison of emission and excitation wavelengths according to the properties set forth above.

Methods for probe labeling are well-known to those of skill in the art and include, for example, chemical and enzymatic methods. Methods for incorporation of reactive chemical groups into oligonucleotides, at specific sites, are well-known to those of skill in the art. Oligonucleotides containing a reactive chemical group, located at a specific site, can be combined with a label attached to a complementary reactive group (e.g., an oligonucleotide containing a nucleophilic reactive group can be reacted with a label attached to an electrophilic reactive group) to couple a label to a probe by chemical techniques. Exemplary labels and methods for attachment of a label to an oligonucleotide are described, for example, in U.S. Pat. No. 5,210,915; Kessler (ed.), Nonradioactive Labeling and Detection of Biomolecules, Springer-Verlag, Berlin, 1992; Kricka (ed.) Nonisotopic DNA Probe Techniques, Academic Press, San Diego, 1992; and Howard (ed.) Methods in Nonradioactive Detection, Appleton & Lange, Norwalk, 1993. Non-specific chemical labeling of an oligonucleotide can be achieved by combining the oligonucleotide with a chemical that reacts, for example, with a particular functional group of a nucleotide base, and simultaneously or subsequently reacting the oligonucleotide with a label. See, for example, Draper et al. (1980) Biochemistry 19:1774-1781. Enzymatic incorporation of label into an oligonucleotide can be achieved by conducting enzymatic modification or polymerization of an oligonucleotide using labeled precursors, or by enzymatically adding label to an already-existing oligonucleotide. See, for example, U.S. Pat. No. 5,449,767. Examples of modifying enzymes include, but are not limited to, DNA polymerases, reverse transcriptases, RNA polymerases, etc. Examples of enzymes that are able to add label to an already-existing oligonucleotide include, but are not limited to, kinases, terminal transferases, ligases, glycosylases, etc.

For use in an amplification assay that involves elevated temperatures, such as PCR, or other procedures utilizing thermostable enzymes, the label will be stable at elevated temperatures. For assays involving polymerization, the label will be such that it does not interfere with the activity of the polymerizing enzyme. Label can be present at the 5' and/or 3' end of the oligonucleotide, and/or may also be present internally. The label can be attached to any of the base, sugar or phosphate moieties of the oligonucleotide, or to any linking group that is itself attached to one of these moieties.

In another preferred embodiment, an oligonucleotide probe according to the invention is a minor grove binding (MGB)-oligonucleotide conjugate. Minor groove binding probes form stable duplexes with single-stranded DNA targets, thus allowing short probes that have a greater discriminatory power to be used in hybridization based assays. Preferably, the MGB moiety is selected from the group consisting of a trimer of 1,2-dihydro-(3H)-pyrrolo[3,2-e]indole-7-carboxylate (CDPI3) and a pentamer of N-methylpyrrole-4-carbox-2-amide (MPC5).

In another embodiment, a guanosine in an NG-specific oligonucleotide of the invention is substituted for a inosine. It has been discovered that substitution of inosine for guanosine in a MGB-oligonucleotide conjugate can enhance hybrid stability. Without wishing to be bound by any particular theory, it is likely that inosine substitution makes the local shape of the minor groove more favourable for interaction with a MGB, thereby increasing the strength of the MGB-minor groove interaction.

In another aspect, the invention provides a method for detecting a NG strain comprising the use of an oligonucleotide according to the invention. In a preferred embodiment, a detection method as provided herein involves polymerase chain reaction (PCR) technology, said method comprising a) providing the DNA of an NG strain or a test sample suspected of containing the DNA of said NG strain; b) amplifying the 5'-untranslated region of an *opa*-gene encompassing nucleotides 57 to 50 located 5' of the *opa* start codon using a pair of nucleic acid amplification primers; and c) detecting the presence of amplification product as an indication of the presence of NG. An oligonucleotide according to the invention can be used as primer in such a PCR assay. The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxynucleotide [The primer must be sufficiently long to prime the synthesis of extension products in the presence of the agent for polymerization.

Detection of the amplification product can in principle be accomplished by any suitable method known in the art. The product may be directly stained or labelled with radioactive labels, antibodies, luminescent dyes, fluorescent dyes, or enzyme reagents. Direct DNA stains include for example intercalating dyes such as acridine orange, SYBR Green, ethidium bromide, ethidium monoazide or Hoechst dyes. Alternatively, the DNA fragments may be detected by incorporation of labelled dNTP bases into the synthesized DNA fragments. (Fluorescent) detection labels that may be associated with nucleotide bases include e.g. fluorescein, cyanine dye or BrdUrd. However, a PCR-based method for detecting NG using an oligonucleotide of the invention preferably involves the detection of the amplified product obtained by the above described DNA amplification reaction by hybridising the reaction product to one or more specific detection probes, wherein an oligonucleotide of the invention is used as detection probe. The term "probe" refers to a single-stranded oligonucleotide sequence that will recognize and form a hydrogen-bonded duplex with a complementary sequence in a target nucleic acid sequence. An oligonucleotide probe of the invention can be used to detect a PCR reaction product comprising the 5'-untranslated region of a *opa* gene encompassing nucleotides 57 to 50 located 5' of the *opa* start codon. Preferably, the amplification primers are designed in such a manner that they flank the target sequence to be detected by the probe (e.g. probe *opa*-2). Of particular use in a NG-detection method of the invention using an oligonucleotide probe of the invention is the primer set consisting of the forward primer *opa*-Fw and the reverse primer *opa*-Rv (see Table 1). This set of primers results in a reaction product of 76 nucleotides, wherein the stretch of nucleotides 57 to 50 located 5' of the *opa* start codon is flanked by about 40 nucleotides at the 5' end and by about 35 nucleotides at the 3' end. The reaction product was successfully detected by probe *opa-*2 of the invention.Preferably, a nucleic acid amplification assay employing an oligonucleotide of the invention (be it as a primer or as a probe) involves real-time quantitative (RQ) PCR analysis. RQ-PCR permits accurate quantitation of PCR products during the exponential phase of the PCR amplification process, which is in full contrast to the classical PCR end point quantitation. Owing tot the real time detection of fluorescent signals during and/or after each subsequent PCR cycle, quantitative PCR data can be obtained in a short period of time and no post-PCR processing is needed, thereby drastically reducing the risk of PCR product contamination. A nucleic acid amplification assay according to the invention can be performed using any type of real time PCR equipment, including theABI PRISM Sequence detection systems, LightCycler & LightCycler 2.0 Instruments by ROCHE DIAGNOSTICS, RapidCycler by Idaho Technology, LightCycler by Idaho Technology, Rotor-Gene, SmartCycler, iCycler & MyiQ Cycler, Mx4000 & Mx3000P, Opticon & Opticon 2, Techne Quantica System, ATC - 901, InSyte Thermal Cycler, Notebookthermal cycler. RQ-PCR technology typically uses ABI Prism 7000, 7700, 7900HT, 7300 or 7500 instruments (TaqMan®) to detect accumulation of PCR products continuously during the PCR process thus allowing easy and accurate quantitation in the early exponential phase of PCR. Some ABI Prism sequence detection systems use fiber optic systems, which connect to each well in a 96-well PCR tray format. A laser light source excites each well and a CCD camera measures the fluorescence spectrum and intensity from each well to generate real-time data during PCR amplification. Other ABI Prism sequence detection systems, such as ABI Prism 7000, use a tungsten-halogen lamp as excitation source, a fresnel lens and a CCD camera to measure the fluorescence. The ABI Prism software examines the fluorescence intensity of reporter and quencher dyes and calculates the increase in normalized reporter emission intensity over the course of the amplification. The results are then plotted versus time, represented by cycle number, to produce a continuous measure of PCR amplification. To provide precise quantification of initial target in each PCR reaction, the amplification plot is examined at a point during the early log phase of product accumulation. This is accomplished by assigning a fluorescence threshold above background and determining the time point at which each sample's amplification plot reaches the threshold (defined as the threshold cycle number or CT).

At present three main types of RQ-PCR techniques can be distinguished; those employing an intercalating dye, those using a so-called hydrolysis (e.g. TaqMan) probe and those using a hybridisation (e.g.LightCycler) probe. In one embodiment, an oligonucleotide according to the invention detection is used as a primer in a PCR assay using the intercalating dye SYBR Green I. This dye can bind to the minor groove of double-stranded DNA, which greatly enhances its fluorescence. During the consecutive PCR cycles, the amount of double stranded PCR product will exponentially increase, and therefore more SYBR Green I dye can bind and emit its fluorescence (at 520 nm). It should be noted that SYBR Green I-based detection of PCR products is not sequence specific and that consequently also non-specifically amplified PCR products and primer dimers will be detected. In addition to SYBR-Green I, also other dyes can be used in non-specific detection systems such as Amplifluor.

In a preferred embodiment, a method of the invention comprises detection and quantitation of an NG strain, using RQ-PCR with a hydrolysis probe according to the invention. This type of RQ-PCR exploits the 5'→3' exonuclease activity of the *Thermus aquaticus* (Taq) polymerase to detect and quantify specific PCR products as the reaction proceeds. The hydrolysis probe, also referred to as TaqMan probe or double-dye oligonucleotide probe, is conjugated with a reporter (R) fluorochrome (e.g. FAM, VIC or JOE) as well as a quencher (Q) fluorochrome (e.g. TAMRA). The quencher fluorochrome absorbs the fluorescence of the reporter fluorochrome as long as the probe is intact. However, upon amplification of the target sequence, i.e. the 5' untranslated region of an *opa*-gene of NG, the hydrolysis probe is displaced and subsequently hydrolysed by the Taq polymerase. This results in the separation of the reporter and quencher fluorochrome and consequently the fluorescence of the reporter fluorochrome becomes detectable. During each consecutive PCR cycle this fluorescence will further increase because of the progressive and exponential accumulation of free reporter fluorochromes.

In yet a further embodiment of the invention, an assay for detecting NG involves RQ-PCR analysis using hybridisation probes. In such an assay two juxtaposed sequence-specific probes are used, one of which is an oligonucleotide according to the invention, wherein one probe is labelled with a donor fluorochrome (e.g. FAM) at the 3' end and the other probe is labelled with an acceptor fluorochrome (e.g. LC Red640, LC Red705) at its 5' end. Both probes should hybridise to closely juxtaposed target sequences on the amplified DNA fragment, thereby bringing the two fluorochromes into close proximity (i.e. within 1-5 nucleotides) such that the emitted light of the donor will excite the acceptor. This results in the emission of fluorescence, which subsequently can be detected during the annealing phase and first part of the extension phase of the PCR reaction. After each subsequent PCR cycle, more hybridisation probes can anneal, resulting in higher fluorescence signals.

In addition to the three main RQ-PCR approaches described above, other types of oligonucleotide probes according to the invention may also be used, including molecular beacons, Scorpions, ResonSense, Hy-Beacon, and Light-up probes.

As said, the primer set consisting of *opa*-Fw and *opa*-Rev in combination with the *opa*-2 detection probe resulted in the detection of aberrant NG strains which were not detected with the *opa*-1 detection probe. When a panel of non-aberrant NG strains was tested with the *opa*-2 probe, i.e. those strains testing positive with the *opa*-1 probe, it appeared that only one out of 21 appeared positive with probe *opa*-2 as well. This result indicates the presence of *opa*-1 and *opa*-2 sequences in that particular strain (data not shown). Thus, in order to detect both non-aberrant and aberrant NG strains in a method of the invention, it is preferred that an oligonucleotide of the invention is used in combination with a probe capable of detecting PCR amplicons of non-aberrant NG strains, such as probe *opa*-1. The threshold cycle (Ct) values show a tenfold higher signal with probe *opa*-1 than with probe *opa*-2.

As is illustrated below, the invention is advantageously used for the diagnosis, of NG in a sample, including clinical samples. Infection with *NG* is known to increase the risk for human immunodeficiency virus (HIV) infection. Odds ratio estimates for increased risk of HIV infection due to previous infection with a sexually transmitted disease (STD) is 3.5 to 9.0 for *NG.* Infection with NG may also be associated with an increased risk of HIV seroconversion. The high incidence rates of both infections, coupled with the prevalence in which they go undiagnosed and/or untreated, highlights the need for greater sexually transmitted disease (STD) screening. With the provision of novel oligonucleotides which recognize NG strains which previously remained undetected, the invention now offers a sensitive, specific, semi-quantitative and reliable assay for the detection of NG in (clinical) specimens and/or for the confirmation of less specific NG tests.

For effective screening and diagnosis, which could lead to prevention and control of NG, attention should be given to screening of single women aged 15-21 and detection in symptomatic patients unlikely to seek diagnosis and treatment.

Also provided herein is a kit for the detection of an NG strain, comprising a pair of nucleic acid amplification primers capable of amplifying a region encompassing nucleotides 57 to 50 located 5' of the NG *opa* start codon and at least one detection probe, wherein at least one primer or detection probe is an oligonucleotide according to the invention. As indicated above, the oligonucleotide may be provided with a detectable label and/or an MGB-moiety. In one embodiment, a kit of the invention comprises an oligonucleotide primer according to the invention. In a preferred embodiment, a kit comprises an oligonucleotide detection probe according to the invention, preferably probe *opa*-2. Most preferably, a kit comprises an oligonucleotide detection probe according to the invention and the primers *opa*-Fw and *opa*-Rv. Preferably, a kit of the invention further comprising detection probe *opa*-1 and/or a polymerase, preferably Taq polymerase.

### LEGENDS TO THE FIGURES

**Figure 1****.** Design of primers and probe. Sequence of *opa*-genes 92 to 16 bases 5' of the start codon retrieved from NCBI database. Light-grey squares indicate position of the primers, the dark-grey squares indicate the position of the probe (sequences as in upper line)
**Figure 2****:** (A) Fluorescent profiles of 10-fold serial dilutions in duplicate from 100 fg to 10 µg/mL of *NG* DNA obtained from ATCC Strain 49226, analysed in the *opa-*based real time PCR using probes *opa*-1 and *opa*-2 and (B) Standard curve calculated from the Ct values: y = - 3,51 * log(x) + 39,341; R² = 0,995.

### EXPERIMENTAL SECTION

To further exemplify the present invention, it is shown below that an oligonucleotide according to the invention is advantageously used for the specific and sensitive detection of NG in clinical samples.

### 1. Materials and methods

**1.1 Panel of 448 *NG* strains.** From September 2002 to April 2003 patients with complaints indicative of gonorrhoea visited the STI (Sexually Transmitted Infections) clinic in Amsterdam, where clinical and epidemiological data were registered and samples were taken. Urethral, cervical, proctal or tonsil specimens were used to inoculate GC-Lect agar plates (Beckton-Dickinson). Culture and determination of *NG* was performed at the Public Health Laboratory in Amsterdam as described (6, 7). In the context of a communal epidemiology study the *NG* strains were typed by PCR-RFLP of the *opa* and *por* genes confirming further that true *NG* strains were used for DNA isolation (18, 21).

**1.2 Panel of 122 clinical Cobas amplicor™ positive samples.** From January 2003 till March 2004 a total of 3957 clinical samples from patients from the region served by Gelre Hospital (Apeldoorn, The Netherlands) were analysed in the Cobas amplicor™ test for the presence of *NG.* 122 samples (3.1%) tested positive for *NG.* These samples, consisting of 36 Urine, 8 urethra, 47 cervix, 29 throat and 2 anal samples (Table 3A), were further analysed in real time 16S rRNA-test and the *opa-*assay.

**1.3 Quality Control for Molecular Diagnostics *NG* 2003 panel.** In order to assess the performance of nucleic acid amplification technologies for detection of *NG,* a proficiency panel was designed by the QCMD Working Party on Sexually Transmitted Diseases (Chair Jurjen Schirm, Groningen, The Netherlands). The panel consisted of lyophilised urine samples. As indicated by QCMD 1.2 ml water was used to dissolve the lyophilised material. Specimens were processed as urines (described below).

### 1.4 Nucleic acid extraction

*Bacterial strains.* For the isolation of nucleic acids from the panel of 448 *NG* strains, DNA was isolated from a few colonies using isopropanol precipitation and followed by dissolving the pellet in 50 µl 10 mM Tris-HCl, pH 8.0 (T10; (6, 20)), and diluted 10.000 times in T10. 5 µl was added to the PCR reaction. For the isolation of nucleic acids from other bacterial strains, bacteria were suspended in TE (1 mM EDTA in 10 mM Tris-HCl buffer pH=8.0) to a suspension of approximately 0.5 McFarland (see: http://biology.fullorton.edu/courses/biol 302/Web/3021abf99/4uant.html#mcfar) and incubated for 15 min. at 100°C. Because of the low Ct values (12-14 indicating high DNA-load) when analysed directly in the real time PCR, 1 in 1000 dilutions in TE were prepared and analysed. 5 µl was added to each PCR.

*Panel of 122 Cobas amplicor*™ *NG positive clinical samples.* One to 1.5 ml of urine was centrifuged 15 min at 13.000 rpm. Supernatant was discarded and approximately 100 µl was left on the pellet. Samples in Amplicor STM and 2-SP medium (media delivered by Roche) were processed directly. DNA was isolated from 100 µl of material using the DNA Isolation Kit III (Bacterial Fungi; Roche Diagnostics Nederland BV Almere, The Netherlands) and the MagnaPure LC Isolation station (Roche Diagnostics Nederland BV Almere, The Netherlands) exactly as described by the manufacturer. The nucleic acids were eluted in a final volume of 100 µl. Isolates were split for Cobas amplicor™, 16S rRNA confirmation tests and *opa*-based *NG* assay, which were all carried out on the same day. 25 µl was added in the Cobas amphcor™, 5 µl was added in the 16S rRNA tests and 10 µl was added to the *opa*-PCR reaction.

*Other clinical samples.* Dry urethra or cervical swabs (plastic minitip swab 185CS01, Copan, AMDS-benelux, Malden, The Netherlands) were placed in 500 µl of TE, incubated for 30 min. at 97°C and centrifuged for 1 min. at 8.000 rpm. 10 µl was used in the PCR. For urine samples: 1 ml was centrifuged at 10.000 rpm for 15 min and supernatant was removed. The remaining pellet was dissolved in 300 µl of TE and incubated for 30 min. at 97°C. 10 µl was used in the PCR.

If inhibition occurred in the PCR (see below), DNA was isolated from 190 µl of sample to which 10 µl of a seal herpesvirus (PhHV) was added using the Qiagen Blood Kit, following manufacturer's guidelines, omitting protease treatment and eluting in 50 µl. 10 µl was used in the PCR reactions.

### 1.5 PCR Inhibition control

To monitor the real-time *NG* detection, a separate PCR was run on all samples to which PhHV-1 was added at a final concentration of approximately 5,000 to 10,000 DNA copies per ml, equivalent to a threshold cycle (Ct) value of approximately 30 (29). If Ct was within range of mean ± 2 standard deviations, the PCR was considered free of inhibition.

**1.6 *Opa*-based *NG* assay.** A 25 µl PCR was performed containing 20 mM TrisHCI pH 8.4, 50 mM KCl, 3 mM MgCl2, (prepared from 10x PCR buffer delivered with Platinum Taq polymerase), 0.75 U Platinum Taq Polymerase (Invitrogen BV, Breda, The Netherlands), 4% glycerol (molecular biology grade; CalBiochem, VWR International B.V., Amsterdam, The Netherlands), 200 µM of each dNTP (Amersham Bioscience, Roosendaal, The Netherlands), 0.5 µl Rox Reference Dye (Invitrogen BV), 150 nM probe *opa*-1, -when indicated- 150 nM probe *opa*-2 (synthesized by Applied Biosystems, Nieuwerkerk a/d IJssel, The Netherlands), 300 nM *opa* Fw primer and 300 nM *opa* Rv primer (Sigma-Genosys Ltd, Haverhill, United Kingdom) and 5 or 10 µl sample (5 µl DNA was used when analysing the 448 *NG* strain panel; 10 µl was used for all the other assays).

ABI Prism sequence detection system 7000 (Applied Biosystems, Nieuwerkerk a/d IJssel, The Netherlands) was used for amplification and detection (2 min. 50°C, 10 min. 95°C, 45 cycli of 15 s 95°C, 60 s 60°C).

**1.7 Cobas amplicor™ test for *NG*.** The Cobas amplicor™ test was performed according to manufacturers instructions.

**1.8 16S rRNA confirmation test** was performed in two independent laboratories.Primers NG16S F: TAT CGG AAC GTA CCG GGT AG and NG16S R: GCT TAT TCT TCA GGT ACC GTC AT were used to amplify a 379 bp fragment, and probes NG16S FL: CGG GTT GTA AAG GAC TTT TGT CAG GGA A-fl and NG16R LC: Red642-AAG GCT GTT GCC AAT ATC GGC GG-p were used to detect the PCR product (Boel E *et al*., manuscript in preparation). 25 µl PCR contained 250 nM of each primer and probe, 4 mM Mg2Cl in 1x LC Mastermix (LC DNA Master Hybridisation Probes kit, Roche Diagnostics Nederland BV Almere, The Netherlands). LightCycler 2.0 (Roche Diagnostics Nederland BV Almere, The Netherlands) was used for amplification and detection (10 min 95°C, 45 cycles of 5 sec 95°C, 10 sec 55°C and 20 sec 72°C; melting curve 20 sec 95°C, 10 sec 35°C, ramp 0.2°C/sec 85°C; cooling 30 sec 40°C)

### 1.9 PhHV-detection. PhHV was detected as described (29).

**1.10 Sequence analysis.** M13-*opa* Fw TGT AAA ACG ACG GCC AGT GTT GAA ACA CCG CCC GG and M13-*opa* Rv CAG GAA ACA GCT ATG ACC CGG TTT GAC CGG TTA AAA AAA GAT primers (300 nM each) were used for amplification. The PCR was carried out as described above. PCR product was purified by adding 4 µl of combined exonuclease I (10U/mL) and shrimp alkaline phosphatase (2 U/µl, USB Corporation, Amersham Bioscience, Roosendaal, The Netherlands) to 20 µl of PCR product, incubation of 15 min. at 37°C, followed by inactivation of 15 min. 80°C (PTC-200 thermocycler, MJ Research, Biozym TC bv, Landgraaf, The Netherlands). Fragments were sequenced using M13 primers. 20 µl reactions contained 5 µl purified PCR product, 4 µl BigDye Terminator Cycle Sequencing Ready Reaction Mix (Applied Biosystems) and 7.5 pmol forward or reverse primer. Twenty-five cycli of 10 sec at 96°C, 5 sec at 50°C and 2.5 min at 60°C were run, and products were purified over Sephadex (G-50 Superfine) before being analysed on a ABI Prism 3700 DNA Analyzer (Applied Biosystems). For each of the 24 "aberrant" *NG* strains, one forward and one reverse sequence was determined.

**1.11 Sensitivity.** *NG* bacteria (ATCC 49226) were resuspended in TE buffer to a suspension of 0.96 McFarland and incubated for 15 min. at 100°C. DNA was isolated from 190 µl of bacterial suspension to which as an internal control 10 µl of PhHV was added using Qiagen Blood Kit, following manufacturer's guidelines, omitting protease treatment. DNA, was eluted in 50 µl and DNA content, determined photospectrometrically (Eppendorf BioPhotometer,) was 18.85 ng/µl (1:1 dilution: A260: 0.192, A280: 0.107, conc. of elute = 18.6 ng/µl, 1:4 dilution: A260: 0.099, A280: 0.056, conc. of elute = 19.1 ng/µl). Ten-fold serial dilutions were made containing 10 µg/mL to 100 fg/mL. 10 µl of each dilution was used for duplicate PCR reactions. One *NG* genome weighs approximately 2.45 fg (2,2 x 10⁹ bp (10) x 665 da/bp x 1.67 x 10-24 g/da).

### 2. Results

**2.1 Primers and probe design.** Sequences covering a conserved region within the 5' untranslated region of the *opa* genes were obtained from the NCBI database. Based on homology, sequences of *NG, N. meningitides* and *N. flava* were retrieved and aligned in Figure 1. Primers and a minor groove binding (MGB) probe *opa*-1 (Table 1) were designed and adapted to TaqMan-standards using Primer Express software (Applied Biosystems). Minor groove binding probes form stable duplexes with single-stranded DNA targets, thus allowing short probes to be used for hybridization based assays.

2.2 Optimisation of the *opa*-based *NG* assay. Of a panel of 448 clinical *NG* strains (see Materials and Methods), 424 generated a positive fluorescent signal in the TaqMan-PCR employing probe *opa*-1, while the fluorescent signal of 24 strains remained undetectable. However, when analysing the PCR products of the 24 aberrant *NG* strains on agarose gel, all 24 showed ample PCR products of the expected size. Apparently the PCR products of the aberrant strains were not detected by probe *opa*-1. Sequencing of the PCR products revealed exactly the same sequence in all 24 strains (indicated in Figure 1). MGB probe *opa*-2 was designed to cover this sequence, and included in the opa-genes based *NG* assay. Because the *opa*-genes are multicopy genes it somewhat surprised us to detect only one sequence in the PCR products of the 24 aberrant *NG* strains. We subsequently analysed DNA from 21 of the non-aberrant *NG* strains from the above panel using the *NG* assay with only probe *opa*-2. One out of 21 appeared positive with probe *opa*-2 as well, indicating the presence of *opa*-1 and *opa*-2 sequences in that particular strain (data not shown). The threshold cycle (Ct) values show a tenfold higher signal with probe *opa*-1 than with probe *opa*-2.

**2.3 Specificity.** Beside the detection of 448 *NG* strains, the specificity of the *NG-*assay was further assessed by testing a panel of non *NG* micro-organisms (Table 2), including DNA from 12 different other *Neisseriaceae.* No signal in the *opa* real time PCR was observed in any of the microorganisms tested using probes *opa*-1 and probe *opa*-2. Thus, the *opa*-based *NG* assay as described is specific for *NG* strains and displays no cross-reactivity with the other Neisseriaceae nor any of the other micro-organisms tested so far.

**2.4 Sensitivity.** Two methods were used to calculate the number of genomes still detectable in the *opa*-assay. DNA was isolated of from *NG* ATCC Strain 49226 as described in Materials and Methods and diluted to undetectable level. Based on the McFarland value of the original bacterial suspension and assuming 1 McFarland to be equivalent to 3 x 10⁸ bacteria/ml, 0.06 bacterial genomes could still be detected in 4 out of 6 reactions. However, the McFarland standard measures turbidity of bacteria and is quite inaccurate as a measure of bacterial quantity. A more precise way of quantifying the number of bacteria is by measuring the DNA content and calculating

the number of bacteria based on genome weight. DNA was spectrophotometrically quantified as described in Materials and Methods and 10-fold serial dilutions ranging from 100 fg to 10 µg DNA per mL were made and amplified in the *opa*-assay. *NG* DNA could be measured linearly over a range of 8 log scales (Figure 2). The PCR efficiency was calculated to be 93% when probes *opa*-1 and *opa*-2 were present in the PCR (efficiency was 98% employing only probe *opa*-1). One fg of *NG* DNA (equivalent to 0.41 *NG* genome) was detectable in 4 out of 6 reactions.

**2.5 Clinical samples.** To test the clinical performance of the *opa*-assay during a 15 months period 122 clinical samples were collected, from a series of 3957 clinical samples, that tested positive in the Cobas amplicor™ test for *NG* (Table 3A). These 122 samples included urine, throat, cervix, urethra and anus swabs. The samples were analysed in two independent laboratories in the 16S rRNA confirmation test and in the *opa*-based *NG* assay. Both laboratories obtained exactly the same results with the 16S rRNA test. Thirty-six samples were found positive and 83 samples were negative in all three tests (Table 3B shows raw data and Table 3C summarizes these results). This is conform the knowledge that the Cobas amplicor™ test produces false positive results that need subsequent confirmation. The remaining three samples that were negative in the 16S rRNA-test were positive in the *opa*-assay. The fact that the three samples showed the highest Ct values in the *opa*-assay (35, 35, and 37) suggested that the discrepancy could be due to a difference in detection level of the 16S rRNA PCR and the *opa*-assay. We therefore analysed a dilution series of *NG* DNA in both assays on the same day. The results of this test revealed a 10 fold difference in sensitivity between the 16S rRNA PCR and the *opa*-PCR in the advantage of the *opa*-PCR (Table 4; the difference is five-fold taking into account the DNA input volume). In this series of 3957 clinical samples the opa-test detected 8% more positives than the 16S rRNA PCR.

**2.6 Quality Control for Molecular Diagnostics (QCMD, Glasgow, UK) panel November 2003.** A QCMD panel was distributed in November 2003. The panel was analysed in the Cobas amplicor™ and 6 samples were sent to two by Roche nominated reference laboratories for *NG* in the Netherlands. In addition, the panel was analysed in the 16S rRNA-test, and in the *opa*-based assay. Results are shown in Table 5. Sample NG03-04 was missed in the Cobas amplicor™ test. Samples NG03-05 and NG03-07 were missed in the confirmation test in both by Roche nominated reference laboratories in the Netherlands. Samples NG03-08 and NG03-09 were missed in one of the two reference laboratories. The *opa*-assay, in contrast, detected all samples that contained *NG.* The threshold cycles of samples NG03-04 and NG03-07 (indicated as Pos (+/-) by QCMD) were 33.2 and 33.5, respectively, indicating a good detection limit of the assay.

**Table 1. Sequences of primers and probes used for real time NG detection.**

| | |
|---|---|
| *opa*-Fw | GTT GAA ACA CCG CCC GG |
| *opa*-Rv | CGG TTT GAC CGG TTA AAA AAA GAT |
| Probe *opa*-1 | CCC TTC AAC ATC AGT GAA A-MGB |
| Probe *opa*-2 | CTT TGA ACC ATC AGT GAA A-MGB |

**Table 2. Reactivity of the opa-based NG assay (probes opa-1 and opa-2) with various species of Neisseria and other micro-organisms.**

| Species | n | Strain Number¹ / Origin | *opa*-assay |
|---|---|---|---|
| *Neisseria cinerea* | 1 | A841390² | Negative |
| *Neisseria denitrificans* | 1 | A841389² | Negative |
| *Neisseria elongata* | 1 | NRBM 901301² | Negative |
| *Neisseria flavescens* | 1 | NRBM 930649² | Negative |
| *Neisseria lactamica* | 2 | NRBM 900295 ^{2, 3} | Negative |
| *Neisseria meningitides* | 11 | ATCC 13102, ^{3, 4} | Negative |
| *Neisseria mucosa* | 2 | 40489², ³ | Negative |
| *Neisseria perflava* | 1 | A841399² | Negative |
| *Neisseria polysaccherea* | 1 | BD02-00484⁵ | Negative |
| *Neisseria sicca* | 1 | A841401² | Negative |
| *Neisseria subflava* | 1 | ³ | Negative |
| *Neisseria subflava var flava* | 1 | NRBM 921185² | Negative |
| | | | |
| *Bacteroides fragilis* | 2 | ATCC 25285, ³ | Negative |
| *Bacteroides vulgatus* | 1 | ATCC 10583 | Negative |
| *Campylobacter jejuni* | 1 | ATCC 11392 | Negative |
| *Chlamydia trachomatis* | 2 | ³ | Negative |
| *Candida albicans* | 2 | ATCC 90028, ³ | Negative |
| *Candida glabrata* | 1 | ATCC 90030 | Negative |
| *Candida krusei* | 1 | ATCC 6258 | Negative |
| *Candida parapsilosis* | 1 | ATCC 90018 | Negative |
| *Corynebacterium aquaticum* | 1 | ⁶ | Negative |
| *Corynebacterium diphteriae mitis* | 1 | SKMM panel 1996 | Negative |
| *Corynebacterium diphteriae belfranti* | 1 | SKMM panel 1996 | Negative |
| *Corynebacterium diphteriae mitis*/*belfanti* | 2 | SKMM panel 2000 | Negative |
| *Corynebacterium diphteriae* | 1 | SKMM panel 2001 | Negative |
| *Corynebacterium ulcerans* | 2 | SKMM panel 2000 | Negative |
| *Cryptococcus neoformans* | 1 | ATCC 90112 | Negative |
| *Enterococcus faecalis* | 1 | ATCC 29212 | Negative |
| *Enterococcus casseliflavus* | 1 | ATCC 700327 | Negative |
| *Escherichia coli* | 3 | ATCC 25922, 35218, ³ | Negative |
| *Gardnerella vaginalis* | 1 | ATCC 14018 | Negative |
| *Haemophilus influenzae* | 3 | ATCC 49247, 49766, 9006, ³ | Negative |
| *Haemophilus parainfluenzae* | 1 | ³ | Negative |
| *Haemophilus ducreyi* | 1 | ³ | Negative |
| *Klebsiella oxytoca* | 1 | ATCC 700324 | Negative |
| *Lactobacillus species* | 1 | ATCC 314 | Negative |
| *Legionalle pneumophila* serogroup 1 | 1 | RMM 220186 | Negative |
| *Moraxella catarrhalis* | 1 | ³ | Negative |
| *M. atlantii* (??) | 1 | ³ | Negative |
| *Moraxella catarrhalis* | 1 | ³ | Negative |
| *Peptostreptococcus magnus* | 1 | ATCC 29328 | Negative |
| *Pseudomonas aeruginosa* | 2 | ATCC 27853, ³ | Negative |
| *Proteus mirabilis* | 1 | ³ | Negative |
| *Salmonella* | 2 | Group B: S36198403, ³ | Negative |
| *Serratia odorifera* | 1 | ATCC 33077 | Negative |
| *Shigella* | 1 | ³ | Negative |
| *Staphylococcus aureus* | 4 | ATCC 25923, 29213, 43300, ³ | Negative |
| *Staphylococcus coagulase neg* | 1 | ³ | Negative |
| *Staphylococcus epidermidis* | 1 | ATCC 12228 | Negative |
| *Staphylococcus marcescens* | 1 | ³ | Negative |
| *Streptococcus pneumoniae* | 3 | ATCC 49619, 6306, ³ | Negative |
| *Streptococcus haemolyticus Group B* | 2 | ⁶ | Negative |
| *MRSA* | 2 | ^{3,8} | Negative |
| *Treponema pallidum* | 1 | ³ | Negative |

| | | | |
|---|---|---|---|
| ¹ATCC, American Type Culture Collection; NRBM, Netherlands Reference Laboratory for Bacterial Meningitis. SKMM, Dutch Organization for Quality Control in Medical Microbiology. ²Amsterdam Medical Center, Academic Medical Center, Dept. Medical Microbiology, Amsterdam ³(6), GG&GD, Municipal Health Service, Amsterdam, The Netherlands. ⁴Detemined by Vitek NHI (Vitek Systems, Inc., Hazelwood, Mo.), confirmed by NRBM. ⁵Special Reference Department for Identification of Bacteria (LIS-BBD), National Institute of Public Health and the Environment (RIVM), The Netherlands. ⁶Determined by API-Coryne (Biomerieux, Boxtel, The Netherlands). ⁷Determined by PathoDx latex Strep Grouping Kit (Diagnostic Products Corporation, Los Angeles, Calif.). ⁸Detemined by Staphaurex« Plus (Remel Inc., Lenexa, KS), confirmed by National Institute of Public Health and the Environment (RIVM), The Netherlands. | | | |

**Table 3.** Evaluation of 16S rRNA *NG* test and *opa*-assay on 122 clinical materials tested positive in the Cobas amplicor™ test for *NG.* The 16S rRNA confirmation test was performed in 2 laboratories: Medical Microbiology and Infectious Diseases, Apeldoorn, Netherlands and a reference laboratory for *Chlamydia trachomatis* and *NG* tests for Roche in the Netherlands. (A) composition of the panel; (B) results of the analysis; (C) summary of the results.
A

| | **Number of specimens** | | |
|---|---|---|---|
| **Material** | | STM medium | 2-SP medium |
| Urine | 36 | | |
| Cervical swab | | 40 | 7 |
| Urethra swab | | 8 | 0 |
| Throat swab | | 13 | 16 |
| Anal swab | | 2 | 0 |

B

| **Number** | **Material** | **Cobas amplicor™** | | | | | ***opa*-based NG assay** | | |
|---|---|---|---|---|---|---|---|---|---|
| | | **1st** | **2nd** | **3rd** | **16S rRNA** | **Result** | **Ct 1** | **Ct 2** | **Result** |
| 3000134 | urethra/ST M | 0,418 | 1,073 | | Undet. | neg | Undet. | Undet. | neg |
| 3000691 | urethra/ST M | 3,874 | 3,864 | 0,003 | pos | POS | 23,18 | 22,88 | POS |
| 3000923 | cervix/STM | >4,000 | >4,000 | | pos | POS | 30.85 | 31,01 | POS |
| 3002317 | Urine | 2,073 | 0,768 | | Undet. | neg | Undet. | Undet. | neg |
| 3002783 | throat/2-SP | 3,176 | 3,133 | | Undet. | neg | Undet. | Undet. | neg |
| 3003334 | cervix/STM | 3,061 | 1,715 | | Undet. | neg | Undet. | Undet. | neg |
| 3003712 | cervix/STM | 0,700 | 1,111 | 1,077 | Undet. | neg | Undet. | Undet. | neg |
| 3003804 | throat/2-SP | 1,741 | 0,617 | 0,241 | Undet. | neg | Undet. | Undet. | neg |
| 3004006 | cervix/STM | 0,023 | 0,000 | 0,256 | Undet. | neg | Undet. | Undet. | neg |
| 3004084 | cervix/STM | 0,013 | 0,413 | 0,428 | Undet. | neg | Undet. | Undet. | neg |
| 3004216 | throat/2-SP | 3,876 | 3,871 | | Undet. | neg | Undet. | Undet. | neg |
| 3004467 | Urine | 3,875 | 3,695 | | Undet. | neg | Undet. | Undet. | neg |
| 3005725 | cervix/STM | 3,697 | 3,695 | | pos | POS | 25,13 | 24,81 | POS |
| 3006301 | cervix/STM | >4,000 | 3,570 | | pos | POS | 30,14 | 29,82 | POS |
| 3007145 | throat/2-SP | 3,700 | 1,737 | | Undet. | neg | Undet. | Undet. | neg |
| 3007162 | Urine | 3,700 | 3,700 | | pos | POS | 18,49 | 18,78 | POS |
| 3007642 | cervix/STM | 2,658 | 2,597 | | Undet. | neg | Undet. | Undet. | neg |
| 3008007 | Urine | >4,000 | 3,865 | | pos | POS | 20,92 | 20,70 | POS |
| 3008130 | cervix/2-SP | 2,729 | 1,755 | | Undet. | neg | Undet. | Undet. | neg |
| 3008789 | cervix/STM | 1,825 | 3,700 | | Undet. | neg | Undet. | Undet. | neg |
| 3008890 | throat/2-SP | 1,274 | 0,551 | | Undet. | neg | Undet. | Undet. | neg |
| 3014038 | Urine | 4,000 | 3,853 | | pos | POS | 20,35 | 20,45 | POS |
| 3014168 | throat/2-sp | 2,899 | 2,943 | | Undet. | neg | Undet. | Undet. | neg |
| 3014426 | Urine | 3,576 | 3,576 | | pos | POS | 22,35 | 22,35 | POS |
| 3014874 | Urine | 3,853 | 4,000 | | Undet. | neg | Undet. | Undet. | neg |
| 3015212 | throat/2-SP | 3,676 | 3,867 | | Undet. | neg | Undet. | Undet. | neg |
| 3015524 | pervix/2-SP | 1,438 | 1,065 | | Undet. | neg | Undet. | Undet. | neg |
| 3015756 | throat/STM | 3,699 | 3,873 | | Undet. | neg | Undet. | Undet. | neg |
| 3016505 | cervix/STM | 1,084 | 0,004 | 2,312 | Undet. | neg | Undet. | Undet. | neg |
| 3016563 | Urine | 3,702 | 3,482 | | pos | POS | 25,75 | 26,37 | POS |
| 3016659 | throat/STM | 1,015 | 0,587 | | Undet. | neg | Undet. | Undet. | neg |
| 3016802 | throat/STM | 3,880 | 3,880 | 1,332 | Undet. | neg | Undet. | Undet. | neg |
| 3017657 | throat/STM | 3,878 | 3,878 | | Undet. | neg | Undet. | Undet. | neg |
| 3017697 | cervix/STM | 3,880 | 2,588 | | Undet. | neg | Undet. | Undet. | neg |
| 3018301 | Urine | >4,000 | >4,000 | | pos | POS | 26,64 | 27,57 | POS |
| 3018340 | Urine | 3,881 | 3,716 | | pos | POS | 17,07 | 16,94 | POS |
| 3018609 | throat/STM | 3,880 | >4,000 | | Undet. | neg | Undet. | Undet. | neg |
| 3019168 | cervix/STM | 3,370 | 3,395 | | pos | POS | 27,40 | 27,68 | POS |
| 3019492 | Urine | 1,018 | 0,429 | 0,366 | Undet. | neg | 34,90 | 35,23 | POS |
| 3019904 | cervix/2-SP | 2,291 | 0,042 | | Undet. | neg | Undet. | Undet. | neg |
| 3020148 | cervix/2-SP | 3,876 | 3,874 | 1,677 | pos | POS | 20,52 | 20,18 | POS |
| 3020175 | Urine | >4,000 | 2,999 | | pos | POS | 27,11 | 28,00 | POS |
| 3021628 | throat/2-SP | 1,925 | 2,663 | | Undet. | neg | Undet. | Undet. | neg |
| 3021792 | cervix/STM | 0,398 | 0,282 | 0,341 | Undet. | neg | Undet. | Undet. | neg |
| 3022405 | urethra/STM | 3,874 | 3,701 | 2,012 | pos | POS | 22,44 | 22,29 | POS |
| 3022410 | cervix/2-SP | 0,239 | 0,281 | 0,810 | Undet. | neg | Undet. | Undet. | neg |
| 3022411 | Urine | 2,687 | 2,670 | | Undet. | neg | Undet. | Undet. | neg |
| 3022616 | Urine | 3,701 | 3,704 | | pos | POS | 14,76 | 15,38 | POS |
| 3022968 | Urine urethra/ST | 3,876 | 3,703 | | pos | POS | 15.74 | 15,45 | POS |
| 3023068 | M | 3,703 | 3,000 | | pos | POS | 19,09 | 18,72 | POS |
| 3023131 | throat/2-SP | 3,700 | 3,700 | | Undet. | neg | Undet. | Undet. | neg |
| 3024817 | throat/2-SP | >4,000 | 3,700 | | Undet. | neg | Undet. | Undet. | neg |
| 3026059 | cervix/STM | 1,585 | 0,923 | | Undet. | neg | Undet. | Undet. | neg |
| 3026466 | Urine | >4,000 | 3,876 | | Undet. | neg | Undet. | Undet. | neg |
| 3026746 | Urine | 2,672 | 2,367 | | Undet. | neg | Undet. | Undet. | neg |
| 3026956 | urethra/STM | 3,873 | >4,000 | | pos | POS | 20,16 | 20,11 | POS |
| 3027264 | cervix/STM | 0,566 | 0,320 | 0,508 | Undet. | neg | Undet. | Undet. | neg |
| 3027747 | cervix/STM | 2,597 | >4,000 | | Undet. | neg | Undet. | Undet. | neg |
| 3028019 | throat/STM | >4,000 | >4,000 | | Undet. | neg | Undet. | Undet. | neg |
| 3028242 | cervix/STM | >4,000 | >4.000 | | Undet. | neg | Undet. | Undet. | neg |
| 3028608 | cervix/STM | 1,628 | 0,164 | 0,756 | Undet. | neg | Undet. | Undet. | neg |
| 3028720 | Urine | 3,871 | 3,574 | | pos | POS | 24,10 | 23,50 | POS |
| 3028885 | Urine | >4,000 | >4,000 | | Undet. | neg | Undet. | Undet. | neg |
| 3028908 | throat/STM | 0,351 | 1,388 | | Undet. | neg | Undet. | Undet. | neg |
| 3029932 | cervix/STM | 3,876 | >4,000 | | Undet. | neg | Undet. | Undet. | neg |
| 3029934 | cervix/STM | 2,687 | 3,479 | | Undet. | neg | Undet. | Undet. | neg |
| 3030124 | Urine | 2,045 | 3,698 | | Undet. | neg | Undet. | Undet. | neg |
| 3030854 | cervix/STM | 1,912 | 0,845 | | Undet. | neg | Undet. | Undet. | neg |
| 3031245 | throat/2-SP | 1,021 | 0,814 | | Undet. | neg | Undet. | Undet. | neg |
| 3031637 | servix/STM | 2,927 | 3,177 | | Undet. | neg | Undet. | Undet. | neg |
| 3033597 | cervix/STM | 1,550 | 0,003 | 0,400 | Undet. | neg | Undet. | Undet. | neg |
| 3034432 | cervix/STM | >4,000 | 3,879 | | pos | POS | 28,38 | 28,83 | POS |
| 3034549 | throat/2-SP | 3,879 | 3,881 | | Undet. | neg | Undet. | Undet. | neg |
| 3034555 | Urine | 3,879 | >4,000 | | pos | PUS | 19.73 | 20,95 | POS |
| 3034615 | cervix/STM | 2,547 | 2,529 | | Undet. | neg | Undet. | Undet. | neg |
| 3035182 | Urine | >4,000 | 3,702 | | pos | POS | 24,38 | 23,74 | POS |
| 3035184 | cervix//STM | >4,000 | 3,878 | | pos | POS | 25,84 | 26,05 | POS |
| 3035191 | urethra/STM | 3,869 | 3,702 | | pos | POS | 22,57 | 23,04 | POS |
| 3035192 | Urine | >4,000 | 1,879 | | pos | POS | 16,44 | 16,53 | POS |
| 3035334 | cervix/STM | 2,579 | 0,002 | 1,400 | neg | leg | Undet. | Undet. | neg |
| 3035774 | throat/STM | 1,511 | 0,872 | 0,582 | Undet. | neg | Undet. | Undet. | neg |
| 3036773 | cervix/2-SP | 0,543 | 1,049 | 0,476 | Undet. | neg | Undet. | Undet. | neg |
| 3037060 | cervix/STM | 0,345 | 2,928 | 0,022 | Undet. | neg | Undet. | Undet. | neg |
| 3037201 | anus/STM | 0,606 | 0,863 | 1,004 | Undet. | neg | Undet. | Undet. | neg |
| 3037203 | throat/STM | 2,924 | 2,707 | | Undet. | neg | Undet. | Undet. | neg |
| 3037284 | anus/STM | 0,612 | 0,943 | 0,295 | Undet. | neg | Undet. | Undet. | neg |
| 3037289 | throat/STM | 0,422 | 1,186 | 0,629 | Undet. | neg | Undet. | Undet. | neg |
| 3037439 | cervix/STM | 1,559 | 1,468 | 0,011 | Undet. | neg | Undet. | Undet. | neg |
| 3037702 | cervix/STM | 1,553 | 1,855 | | Undet. | neg | 34,59 | 34,92 | POS |
| 3038009 | Urine | >4,000 | 1,237 | | pos | POS | 18,29 | 18,32 | POS |
| 3038033 | Urine | 3,710 | >4,000 | | pos | POS | 25,73 | 24,41 | POS |
| 3036897 | urine | 3,883 | 3,882 | | pos | POS | 19,38 | 21,28 | POS |
| 3030191 | urethra/STM | 3,702 | 3,698 | | pos | POS | 22,12 | 22,68 | POS |
| 3021712 | throat/STM | 0,884 | 2,44 | 2,034 | Undet. | neg | Undet. | Undet. | neg |
| 3021756 | urine | 3,691 | 3,873 | | pos | POS | 18,72 | 19,06 | POS |
| 3021769 | urine | 3,867 | 3,873 | | pos | POS | 29,06 | 29,20 | POS |
| 3021202 | throat/STM | 0,407 | 0,372 | 0,346 | Undet. | neg | Undet. | Undet. | neg |
| 3018355 | urine | 2,908 | 2,504 | | Undet. | neg | Undet. | Undet. | neg |
| 3014292 | urine | 2,392 | 2,409 | | Undet. | neg | Undet. | Undet. | neg |
| 3002538 | urine | >4,000 | 3,873 | | pos | POS | 16,16 | 16,12 | POS |
| 3002536 | cervix/STM | 3,686 | 2,832 | | Undet. | neg | Undet. | Undet. | neg |
| 4001319 | cervix/STM | 3,876 | >4,000 | | Undet. | neg | 36,20 | 37,97 | POS |
| 4001329 | urine | 1,187 | 1,008 | 0,810 | Undet. | neg | Undet. | Undet. | neg |
| 4001343 | urine | >4,000 | >4,000 | | pos | POS | 28,09 | 28,54 | POS |
| 4001818 | throat/2-SP | 3,881 | 3,702 | | Undet. | neg | Undet. | Undet. | neg |
| 4002020 | urine | >4,000 | 3,099 | | Undet. | neg | Undet. | Undet. | neg |
| 4002021 | cervix/2-SP | >4,000 | >4,000 | | Undet. | neg | Undet. | Undet. | neg |
| 4002056 | urethra/STM | >4,000 | 3.878 | | Undet. | | Undet. | Undet. | neg |
| 4002770 | cervix/STM | 0,337 | 0,277 | 0,135 | Undet. | neg | Undet. | Undet. | neg |
| 4003263 | cervix/STM | 2,24 | 0,714 | 2,309 | Undet. | neg | Undet. | Undet. | neg |
| 4003420 | cervix/STM | 3,708 | >4,000 | | pos | POS | 29,47 | 29,68 | POS |
| 4004017 | cervix/STM | >4,000 | >4,000 | | Undet. | neg | Undet. | Undet. | neg |
| 4004160 | cervix/STM | 0,278 | 0,203 | 0,288 | Undet. | neg | Undet. | Undet. | neg |
| 4004282 | throat/2-SP | >4,000 | >4,000 | | Undet. | neg | Undet. | Undet. | neg |
| 4005083 | throat/STM | 2,123 | 3,036 | | Undet. | neg | Undet. | Undet. | neg |
| 4005301 | cervix/STM | 0,212 | 1,015 | 0,625 | Undet. | neg | Undet. | Undet. | neg |
| 4005658 | urine | 1,838 | 3,104 | | Undet. | neg | Undet. | Undet. | neg |
| 4006517 | urine | 3,886 | 3,882 | | pos | POS | 19,55 | 20,02 | POS |
| 4006379 | cervix/STM | 3,886 | 3,882 | | Undet. | neg | Undet. | Undet. | neg |
| 4006381 | cervix/STM | 3,71 | >4,000 | | Undet. | neg | Undet. | Undet. | neg |
| 4006589 | throat/2-SP | 3,882 | >4,000 | | Undet. | neg | Undet. | Undet. | neg |
| 4006824 | throat/2-SP | 3,579 | 0,665 | 3,283 | Undet. | neg | Undet. | Undet. | neg |

C

| Material | Assay | *Opa*-assay | | |
|---|---|---|---|---|
| | | Result | Neg | Pos |
| Urine | 16S rRNA* | Neg | 13 | 1 |
| | | Pos | 0 | 22 |
| Cervical swab | 16S rRNA* | Neg | 37 | 2 |
| | | Pos | 0 | 8 |
| Urethra swab | 16S rRNA* | Neg | 0 | 6 |
| | | Pos | 29 | 0 |
| Throat swab | 16S rRNA* | Neg | 2 | 0 |
| | | Pos | 2 | 0 |
| Anal swab | 16S rRNA* | Neg | 83 | 3 |
| Total | 16S rRNA* | Pos | 0 | 36 |

| | | | | |
|---|---|---|---|---|
| *Exactly the same results were obtained in the two independent laboratories. | | | | |

**Table 4. Comparison of sensitivity of the 16S rRNA-test and the opa-assay. Numbers correspond to threshold cycle numbers (CT).**

| DNA in PCR (fg/µl) | Ct 16S rRNA-test | Ct *opa*-assay |
|---|---|---|
| 10.000 | 27.06 | 24.3 ± 0.0 |
| 1.000 | 32.17 | 28.4 ± 0.0 |
| 100 | 36.22 | 32.0 ± 0.1 |
| 10 | >41.00 | 35.9 ± 0.3 |
| 1 | undetectable | 42.4 |

**Table 5. Evaluation of the QCMD NG panel November 2003.**

| **QCMD Code** | **Cobas amplicor™** | **Ref. Lab 1** | **Ref. Lab 2** | **16S rRNA-test** | ***Opa*-assay** | | **QCMD Result** | **% correct results** |
|---|---|---|---|---|---|---|---|---|
| | | | | | **Result** | **Ct** | | |
| NG03-01 | 0.005 | | | Neg | Neg | Undet. | Neg | 100 % |
| NG03-02 | 0.004 | | | Neg | Neg | Undet. | Neg | 100 % |
| NG03-03 | 3.873 | Pos | Pos | Pos | Pos | 28.4 | Pos (++) | 97 % |
| NG03-04 | 0.006 | | | Neg | Pos | 33.2 | Pos (+/-) | 48 % |
| NG03-05 | 2.541 | Neg | Neg | Neg | Pos | 31.2 | Pos (+) | 92 % |
| NG03-06 | 0.004 | | | Neg | Neg | Undet. | Neg | 98 % |
| NG03-07 | 2.157 | Neg | Neg | Neg | Pos | 33.5 | Pos (+/-) | 53 % |
| NG03-08 | 3.873 | Pos | Neg | Pos | Pos | 30.8 | Pos (+) | 90 % |
| NG03-09 | 3.873 | Neg | Pos | Pos | Pos | 26.5 | Pos (+) | 81 % |
| NG03-10 | >4,000 | Pos | Pos | Pos | Pos | 28.1 | Pos (++) | 95 % |

| | | | | | | | | |
|---|---|---|---|---|---|---|---|---|
| >4,000: Signal above 4.000 | | | | | | | | |

### REFERENCES

1. Akduman, D., J. M. Ehret, K. Messina, S. Ragsdale, and F. N. Judson. 2002. Evaluation of a strand displacement amplification assay (BD ProbeTec-SDA) for detection of Neisseria gonorrhoeae in urine specimens. J Clin Microbiol 40:281-3.
2. Bassiri, M., P. A. Mardh, and M. Domeika. 1997. Multiplex AMPLICOR PCR screening for Chlamydia trachomatis and Neisseria gonorrhoeae in women attenting non-sexually transmitted disease clinics. The European Chlamydia Epidemiology Group. J Clin Microbiol 35:2556-60.
3. Bessen, D., and E. C. Gotschlich. 1986. Interactions of gonococci with HeLa cells: attachment, detachment, replication, penetration, and the role of protein II. Infect Immun 54:154-60.
4. Bhat, K. S., C. P. Gibbs, O. Barrera, S. G. Morrison, F. Jahnig, A. Stern, E. M. Kupsch, T. F. Meyer, and J. Swanson. 1991. The opacity proteins of Neisseria gonorrhoeae strain MS11 are encoded by a family of 11 complete genes. Mol Microbiol 5:1889-901.
5. Bos, M. P., D. Hogan, and R. J. Belland. 1997. Selection of Opa+ Neisseria gonorrhoeae by limited availability of normal human serum. Infect Immun 65:645-50.
6. Bruisten, S. M., G. T. Noordhoek, A. J. C. van den Brule, B. Duim, C. H. E. Boel, K. El-Faouzi, R. du Maine, S. Mulder, D. Luijt, and J. Schirm. 2004. Multicenter Validation of the cppB Gene as a PCR Target for Detection of Neisseria gonorrhoeae. J Clin Microbiol In Press**.**
7. Buimer, M., G. J. van Doornum, S. Ching, P. G. Peerbooms, P. K. Plier, D. Ram, and H. H. Lee. 1996. Detection of Chlamydia trachomatis and Neisseria gonorrhoeae by ligase chain reaction-based assays with clinical specimens from various sites: implications for diagnostic testing and screening. J Clin Microbiol 34:2395-400.
8. Chen, T., R. J. Belland, J. Wilson, and J. Swanson. 1995. Adherence of pilus- Opa+ gonococci to epithelial cells in vitro involves heparan sulfate. J Exp Med 182:511-7.
9. Ching, S., H. Lee, E. W. Hook, 3rd, M. R. Jacobs, and J. Zenilman. 1995. Ligase chain reaction for detection of Neisseria gonorrhoeae in urogenital swabs. J Clin Microbiol 33:3111-4.
10. Dempsey, J. A., W. Litaker, A. Madhure, T. L. Snodgrass, and J. G. Cannon. 1991. Physical map of the chromosome of Neisseria gonorrhoeae FA1090 with locations of genetic markers, including opa and pil genes. J Bacteriol 173:5476-86.
11. Farrell, D. J. 1999. Evaluation of AMPLICOR Neisseria gonorrhoeae PCR using cppB nested PCR and 16S rRNA PCR. J Clin Microbiol 37:386-90.
12. Hook, E. W. r., and H. H. Handsfield. 1990. Gonococcal infections in the adult. In K. K. H. e. al (ed.), Sexually transmitted diseases. McGraw-Hill, New York.
13. Katz, A. R., P. V. Effler, R. G. Ohye, B. Brouillet, M. V. Lee, and P. M. Whiticar. 2004. False-positive gonorrhea test results with a nucleic acid amplification test: the impact of low prevalence on positive predictive value. Clin Infect Dis 38:814-9.
14. Kehl, S. C., K. Georgakas, G. R. Swain, G. Sedmak, S. Gradus, A. Singh, and S. Foldy. 1998. Evaluation of the abbott LCx assay for detection of Neisseria gonorrhoeae in endocervical swab specimens from females. J Clin Microbiol 36:3549-51.
15. Kupsch, E. M., B. Knepper, T. Kuroki, I. Heuer, and T. F. Meyer. 1993. Variable opacity (Opa) outer membrane proteins account for the cell tropisms displayed by Neisseria gonorrhoeae for human leukocytes and epithelial cells. Embo J 12:641-50.
16. Makino, S., J. P. van Putten, and T. F. Meyer. 1991. Phase variation of the opacity outer membrane protein controls invasion by Neisseria gonorrhoeae into human epithelial cells. Embo J 10:1307-15.
17. Martin, D. H., C. Cammarata, B. Van Der Pol, R. B. Jones, T. C. Quinn, C. A. Gaydos, K. Crotchfelt, J. Schachter, J. Moncada, D. Jungkind, B. Turner, and C. Peyton. 2000. Multicenter evaluation of AMPLICOR and automated COBAS AMPLICOR CT/NG tests for Neisseria gonorrhoeae. J Clin Microbiol 38:3544-9.
18. O'Rourke, M., C. A. Ison, A. M. Renton, and B. G. Spratt. 1995. Opa-typing: a high-resolution tool for studying the epidemiology of gonorrhoea. Mol Microbiol 17:865-75.
19. Palladino, S., J. W. Pearman, I. D. Kay, D. W. Smith, G. B. Harnett, M. Woods, L. Marshall, and J. McCloskey. 1999. Diagnosis of Chlamydia trachomatis and Neisseria gonorrhoeae. Genitourinary infections in males by the Amplicor PCR assay of urine. Diagn Microbiol Infect Dis 33:141-6.
20. Peerbooms, P. G., M. N. Engelen, D. A. Stokman, B. H. van Benthem, M. L. van Weert, S. M. Bruisten, A. van Belkum, and R. A. Coutinho. 2002. Nasopharyngeal carriage of potential bacterial pathogens related to day care attendance, with special reference to the molecular epidemiology of Haemophilus influenzae. J Clin Microbiol 40:2832-6.
21. Rahman, M., A. Alam, K. Nessa, S. Nahar, D. K. Dutta, L. Yasmin, S. Monira, Z. Sultan, S. A. Khan, and M. J. Albert. 2001. Treatment failure with the use of ciprofloxacin for gonorrhea correlates with the prevalence of fluoroquinolone-resistant Neisseria gonorrhoeae strains in Bangladesh. Clin Infect Dis 32:884-9.
22. Roche Diagnostic Systems Inc. 1999. Amplicor® Chlamydia trachomatis/Neisseria gonorrhoeae (CT/NG) test package insert., Roche Diagnostic Systems Inc. Branchburg, N.J.
23. Schmale, J. D., J. E. Martin, Jr., and G. Domescik. 1969. Observations on the culture diagnosis of gonorrhea in women. Jama 210:312-4.
24. Smith, K. R., S. Ching, H. Lee, Y. Ohhashi, H. Y. Hu, H. C. Fisher, 3rd, and E. W. Hook, 3rd. 1995. Evaluation of ligase chain reaction for use with urine for identification of Neisseria gonorrhoeae in females attending a sexually transmitted disease clinic. J Clin Microbiol 33:455-7.
25. Stern, A., M. Brown, P. Nickel, and T. F. Meyer. 1986. Opacity genes in Neisseria gonorrhoeae: control of phase and antigenic variation. Cell 47:61-71.
26. Swanson, J. 1978. Studies on gonococcus infection. XII. Colony color and opacity varienats of gonococci. Infect Immun 19:320-31.
27. Toleman, M., E. Aho, and M. Virji. 2001. Expression of pathogen-like Opa adhesins in commensal Neisseria: genetic and functional analysis. Cell Microbiol 3:33-44.
28. Van Der Pol, B., D. V. Ferrero, L. Buck-Barrington, E. Hook, 3rd, C. Lenderman, T. Quinn, C. A. Gaydos, J. Lovchik, J. Schachter, J. Moncada, G. Hall, M. J. Tuohy, and R. B. Jones. 2001. Multicenter evaluation of the BDProbeTec ET System for detection of Chlamydia trachomatis and Neisseria gonorrhoeae in urine specimens, female endocervical swabs, and male urethral swabs. J Clin Microbiol 39:1008-16.
29. van Doornum, G. J., J. Guldemeester, A. D. Osterhaus, and H. G. Niesters. 2003. Diagnosing herpesvirus infections by real-time amplification and rapid culture. J Clin Microbiol 41:576-80.
30. Van Dyck, E., H. Smet, L. Van Damme, and M. Laga. 2001. Evaluation of the Roche Neisseria gonorrhoeae 16S rRNA PCR for confirmation of AMPLICOR PCR-positive samples and comparison of its diagnostic performance according to storage conditions and preparation of endocervical specimens. J Clin Microbiol 39:2280-2.
31. van Putten, J. P., and S. M. Paul. 1995. Binding of syndecan-Like cell surface proteoglycan receptors is required for Neisseria gonorrhoeae entry into human mucosal cells. Embo J 14:2144-54.
32. Weel, J. F., C. T. Hopman, and J. P. van Putten. 1991. In situ expression and localization of Neisseria gonorrhoeae opacity proteins in infected epithelial cells: apparent role of Opa proteins in cellular invasion. J Exp Med 173:1395-405.

## Claims

1. A *Neisseria gonorrhoeae* -specific oligonucleotide, comprising a nucleotide sequence comprising the sequence 5'-TTTGAACC-3', or its complement, capable of hybridising to the 5'-untranslated region of an *opa*-gene of NG or its complement, wherein said oligonucleotide is 12-40 nucleotides, preferably 14-30, more preferably 16-25, most preferably 18-22 nucleotides in length, with the proviso that said sequence is not 5'-tcagtgatggttcaaagttc-3.

2. Oligonucleotide according to claim 1 comprising the nucleotide sequence 5'-CTTTGAACCATCAGTGAAA-3' or its complement (probe *opa*-2).

3. Oligonucleotide according to claim 1 or 2, comprising one or more detectable labels, preferably a chromophore or fluorescent label, more preferably a label selected from the group consisting of 6-FAM, HEX, JOE, TET, ROX, TAMRA, Fluorescein, Cy3, Cy5, Cy5.5, Texas Red, Rhodamine, Rhodamine Green, Rhodamine Red, 6-CarboxyRhodamine 6G, Oregon Green 488, Oregon Green 500, Oregon Green 514 DABCYL, BHQ-1 and BHQ-2.

4. Oligonucleotide according to any one of claims 1 to 3, wherein said oligonucleotide is a minor grove binding (MGB)-oligonucleotide conjugate, preferably wherein MGB is selected from the group consisting of a trimer of 1,2-dihydro-(3H)-pyrrolo[3,2-e]indole-7-carboxylate (CDPI3) and a pentamer of N-methylpyrrole4-carbox-2-amide (MPC5).

5. A method for detecting a *Neisseria gonorrhoeae* strain comprising the use of an oligonucleotide according to any one of claims 1 to 4, wherein said method preferably comprises nucleic acid amplification.

6. Method according to claim 5 involving polymerase chain reaction (PCR) technology, preferably real-time quantitative (RQ-PCR) technology, said method comprising:
a) providing the DNA of a NG strain or a test sample suspected of containing the DNA of said NG strain;
b) amplifying the 5'-untranslated region of a opa-gene encompassing nucleotides 57 to 50 located 5' of the *opa* start codon using a pair of nucleic acid amplification primers; and
c) detecting the presence of amplification product an indication of the presence of NG, preferably using at least one detection probe capable of hybridising to the amplification product;
wherein at least one primer or at least one detection probe is an oligonucleotide according to any one of claims 1 to 4.

7. Method according to claim 6, wherein said primer pair comprises *opa* Fw and *opa* Rv shown in Table 1.

8. Method according to claim 7, wherein said detection probe has the nucleotide sequence 5'-CTT TGA ACC ATC AGT GAA A-3'.

9. Method according to claim 8, wherein an additional detection probe is used, preferably wherein said detection probe has the nucleotide sequence 5'-CCC TTC AAC ATC AGT GAA A-3'.

10. Use of a method according to any one of claims 6 to 9 for the detection, preferably the quantitative detection, of the presence of *Neisseria gonorrhoeae* in a clinical sample.

11. A kit for the detection of a *Neisseria gonorrhoeae* strain, comprising a pair of nucleic acid amplification primers capable of amplifying a region encompassing nucleotides 57 to 50 located 5' of the *Neisseria gonorrhoeae opa* gene start codon and at least one detection probe, wherein at least one primer and/or detection probe is an oligonucleotide according to any one of claims 1-4, optionally further comprising a polymerase, preferably Taq polymerase.

12. Kit according to claim 11, comprising the primers *opa* Fw and *opa* Rv and detection probe *opa*-2, optionally further comprising detection probe *opa*-1.

## Patentansprüche

1. *Neisseria-gonorrhoeae*-spezifisches Oligonucleotid, das eine Nucleotidsequenz, die die Sequenz 5'-TTTGAACC-3' umfasst, oder deren Komplement umfasst, die bzw. das mit dem 5'-untranslatierten Bereich eines opa-Gens von *Neisseria gonorrhoeae* oder dessen Komplement hybridisieren kann, wobei das Oligonucleotid eine Länge von 12-40 Nucleotiden, vorzugsweise 14-30, besonders bevorzugt 16-25, am meisten bevorzugt 18-22 Nucleotiden hat, mit der Maßgabe, dass es sich bei der Sequenz nicht um 5'-tcagtgatggttcaaagttc-3' handelt.

2. Oligonucleotid gemäß Anspruch 1, das die Nucleotidsequenz 5'-CTTTGAACCATCAGTGAAA-3' oder deren Komplement (Sonde opa-2) umfasst.

3. Oligonucleotid gemäß Anspruch 1 oder 2, das einen oder mehrere nachweisbare Marker umfasst, vorzugsweise ein Chromophor oder einen Fluoreszenzmarker, besonders bevorzugt einen Marker, der aus der Gruppe ausgewählt ist, die aus 6-FAM, HEX, JOE, TET, ROX, TAMRA, Fluorescein, Cy3, Cy5, Cy5.5, Texas Red, Rhodamin, Rhodamine Green, Rhodamine Red, 6-CarboxyRhodamine 6G, Oregon Green 488, Oregon Green 500, Oregon Green 514, DABCYL, BHQ-1 und BHQ-2 besteht.

4. Oligonucleotid gemäß einem der Ansprüche 1 bis 3, wobei das Oligonucleotid ein MGB(minor groove binder)-Oligonucleotid-Konjugat ist, wobei der MGB vorzugsweise aus der Gruppe ausgewählt ist, die aus einem Trimer von 1,2-Dihydro(3H)pyrrolo[3,2-e]indol-7-carboxylat (CDPI3) und einem Pentamer von N-Methylpyrrol-4-carbox-2-amid (MPC5) besteht.

5. Verfahren zum Nachweisen eines *Neisseria-gonorrhoeae-Stamms,* das die Verwendung eines Oligonucleotids gemäß einem der Ansprüche 1 bis 4 umfasst, wobei das Verfahren vorzugsweise eine Nucleinsäureamplifikation umfasst.

6. Verfahren gemäß Anspruch 5, das die Technik der Polymerase-Kettenreaktion (PCR), vorzugsweise die Technik der quantitativen Echtzeit-PCR (RQ-PCR), beinhaltet, wobei das Verfahren Folgendes umfasst:
a) Bereitstellen der DNA eines *Neisseria-gonorrhoeae*-Stamms oder einer Testprobe, von der man annimmt, dass sie die DNA des *Neisseria-gonorrhoeae*-Stamms enthalten könnte;
b) Amplifizieren des 5'-untranslatierten Bereichs eines opa-Gens, der die Nucleotide 57 bis 50 umfasst, die sich 5' des opa-Startcodons befinden, wobei man ein Paar von Nucleinsäure-Amplifikationsprimern verwendet; und
c) Nachweisen der Anwesenheit von Amplifikationsprodukt als Hinweis auf die Anwesenheit von *Neisseria gonorrhoeae,* wobei man vorzugsweise wenigstens eine Nachweissonde verwendet, die mit dem Amplifikationsprodukt hybridisieren kann, wobei wenigstens ein Primer oder wenigstens eine Nachweissonde ein Oligonucleotid gemäß einem der Ansprüche 1 bis 4 ist.

7. Verfahren gemäß Anspruch 6, wobei das Primerpaar opa Fw und opa Rv umfasst, die in Tabelle 1 gezeigt sind.

8. Verfahren gemäß Anspruch 7, wobei die Nachweissonde die Nucleotidsequenz 5'-CTT TGA ACC ATC AGT GAA A-3' aufweist.

9. Verfahren gemäß Anspruch 8, wobei eine zusätzliche Nachweissonde verwendet wird, wobei die Nachweissonde vorzugsweise die Nucleotidsequenz 5'-CCC TTC AAC ATC AGT GAA A-3' aufweist.

10. Verwendung eines Verfahrens gemäß einem der Ansprüche 6 bis 9 zum Nachweis, vorzugsweise zum quantitativen Nachweis, der Anwesenheit von *Neisseria gonorrhoeae* in einer klinischen Probe.

11. Kit zum Nachweis eines *Neisseria-gonorrhoeae*-Stamms, umfassend ein Paar von Nucleinsäure-Amplifikationsprimern, das einen Bereich, der die Nucleotide 57 bis 50 umfasst, die sich 5' des *Neisseria-gonorrhoeae*-opa-Startcodons befinden, amplifizieren kann, und wenigstens eine Nachweissonde, wobei wenigstens ein Primer und/oder die Nachweissonde ein Oligonucleotid gemäß einem der Ansprüche 1 bis 4 ist, gegebenenfalls weiterhin umfassend eine Polymerase, vorzugsweise Taq-Polymerase.

12. Kit gemäß Anspruch 11, umfassend die Primer opa Fw und opa Rv und die Nachweissonde opa-2, gegebenenfalls weiterhin umfassend die Nachweissonde opa-1.

## Revendications

1. Oligonucléotide spécifique à *Neisseria gonorrhoeae,* comprenant une séquence de nucléotides comprenant la séquence 5'-TTTGAACC-3', ou son complément, capable de s'hybrider à la région non traduite 5' d'un gène *opa* de NG ou à son complément, où ledit oligonucléotide a une longueur de 12-40 nucléotides, de préférence 14-30, de manière davantage préférée 16-25, de la manière la plus préférée entre toutes 18-22 nucléotides, à condition que ladite séquence ne soit pas 5'-tcagtgatggttcaaagttc-3'.

2. Oligonucléotide selon la revendication 1, comprenant la séquence de nucléotides 5'-CTTTGAACCATCAGTGAAA-3' ou son complément (sonde *opa*-2).

3. Oligonucléotide selon la revendication 1 ou 2, comprenant un ou plusieurs marqueurs détectables, de préférence un chromophore ou un marqueur fluorescent, de manière davantage préférée un marqueur sélectionné dans le groupe consistant en 6-FAM, HEX, JOE, TET, ROX, TAMRA, fluorescéine, Cy3, Cy5, Cy5.5, Texas Red, Rhodamine, Rhodamine Green, Rhodamine Red, 6-carboxyrhodamine 6G, Oregon Green 488, Oregon Green 500, Oregon Green 514, DABCYL, BHQ-1 et BHQ-2.

4. Oligonucléotide selon l'une quelconque des revendications 1 à 3, où ledit oligonucléotide est un conjugué ligand du sillon mineur (MGB)-oligonucléotide, dans lequel de préférence le MGB est sélectionné dans le groupe consistant en un trimère de 1,2-dihydro-(3H)-pyrrolo[3,2-e]indole-7-carboxylate (CDP13) et un pentamère de N-méthylpyrrole-4-carbox-2-amide (MPC5).

5. Procédé pour détecter une souche de *Neisseria gonorrhoeae,* comprenant l'utilisation d'un oligonucléotide selon l'une quelconque des revendications 1 à 4, où ledit procédé comprend de préférence une amplification d'acide nucléique.

6. Procédé selon la revendication 5, impliquant une technologie de réaction en chaîne par polymérase (PCR), de préférence une technologie quantitative en temps réel (RQ-PCR), ledit procédé consistant à :
a) mettre à disposition l'ADN d'une souche NG ou un échantillon de test suspecté de contenir l'ADN de ladite souche NG ;
b) amplifier la région non traduite 5' d'un gène *opa* englobant les nucléotides 57 à 50 situés en 5' du codon d'initiation de *opa* en utilisant une paire d'amorces d'amplification d'acide nucléique ; et
c) détecter la présence d'un produit d'amplification en tant qu'indication de la présence de NG, en utilisant de préférence au moins une sonde de détection capable de s'hybrider au produit d'amplification ;
où au moins une amorce ou au moins une sonde de détection est un oligonucléotide selon l'une quelconque des revendications 1 à 4.

7. Procédé selon la revendication 6, dans lequel ladite paire d'amorces comprend *opa* Fw et *opa* Rv montrées dans le Tableau 1.

8. Procédé selon la revendication 7, dans lequel ladite sonde de détection possède la séquence de nucléotides 5'-CTT TGA ACC ATC AGT GAA A-3'.

9. Procédé selon la revendication 8, dans lequel une sonde de détection supplémentaire est utilisée, de préférence où ladite sonde de détection possède la séquence de nucléotides 5'-CCC TTC AAC ATC AGT GAA A-3'.

10. Utilisation d'un procédé selon l'une quelconque des revendications 6 à 9 pour la détection, de préférence la détection quantitative, de la présence de *Neisseria gonorrhoeae* dans un échantillon clinique.

11. Kit pour la détection d'une souche de *Neisseria gonorrhoeae,* comprenant une paire d'amorces d'amplification d'acide nucléique capable d'amplifier une région englobant les nucléotides 57 à 50 situés en 5' du codon d'initiation du gène *opa* de *Neisseria gonorrhoeae* et au moins une sonde de détection, où au moins une amorce et/ou sonde de détection est un oligonucléotide selon l'une quelconque des revendications 1 à 4, comprenant facultativement en outre une polymérase, de préférence une Taq polymérase.

12. Kit selon la revendication 11, comprenant les amorces *opa* Fw et *opa* Rv et la sonde de détection *opa*-2, comprenant facultativement en outre une sonde de détection *opa*-1.
